# EUROPÄISCHE PATENTANMELDUNG

(11) **EP 0 956 867 A1**
(43) Veröffentlichungstag der Anmeldung: **17.11.1999**
(21) Anmeldenummer: 98108560.8
(22) Anmeldetag: 12.05.1998
(51) Int. Cl.: A61K 45/06, A61K 31/70, A61K 31/35, A61K 35/78

(54) **Verwendung von Flavonoidglykosiden, Gerbstoffen und Mikroorganismen zur Therapie und Prophylaxe des Diabetes mellitus**

(71) Anmelder: Liebel, Franz-Peter, Dr., D-33619 Bielefeld (DE)
(72) Erfinder: Liebel, Franz-Peter, Dr., D-33619 Bielefeld (DE)

(57) **Zusammenfassung**

Es wird ein pathogenetisches Konzept der Insulinresistenz und des darauf basierenden Diabetes mellitus vorgelegt.

Das Konzept geht aus von einem Permeabilitätsdefekt der Dünndarmmukosa, der zu einer Inaktivierung der Insulinrezeptoren im Gefäßendothel und im weiteren zu einer Insulinmangelsituation an den Zielzellen führt.

Mit verschiedenen Substanzen wird gezielt in die Pathogenese der Insulinresistenz eingegriffen. Gebessert werden dabei auch sogenannte diabetische Begleiterkrankungen.

## Beschreibung

### 1. Verwendbarkeit der Erfindung

Das hier vorgestellte pathogenetische Konzept zeigt im einzelnen auf, welche pathophysiologischen Zustände und welche Mechanismen zu einer Etablierung einer Insulinresistenz führen.

Es ist unmittelbar einsichtig, daß man mit einem solchen Konzept in der Lage ist, mit geeigneten Substanzen ursächlich die Insulinresistenz zu therapieren.

Darüberhinaus werden durch die Therapie der Insulinresistenz zwangsläufig solche Erkrankungen gebessert, in deren pathogenetischem Ablaufschema die Insulinresistenz oder zur Insulinresistenz führende pathophysiologische Zustände erscheinen.

### 2. Stand der Technik

Die derzeitigen Modelle zur Pathogenese der Insulinresistenz sind falsch oder treffen neben das eigentliche Problem, das darin besteht, die Entwicklungsschritte bis hin zu einer Insulinresistenz aufzuzeigen.

Vergleiche Berger, H.: Diabetes mellitus. Urban & Schwarzenberg, 1995, S.381. Ausgehend von Hyperinsulinämie oder Hyperglykämie wird die Insulinresistenz in einem Kausalschritt erreicht; der Pathomechanismus bleibt unaufgeklärt.

Vergleiche Mehnert, H. et al. (Hrsg.): Diabetologie in Klinik und Praxis, Georg Thieme Verlag, 1994, S.56. Hier wird die Insulinresistenz gleich als primärer Defekt des Zielgewebes postuliert.

### 3. Vorteile des Verfahrens

Die Behandlung eines auf einer Insulinresistenz und einer Erschöpfung der β-Zellen basierenden Diabetes mellitus mit Sulfonylharnstoffen oder Biguaniden ist bereits vorn Grundsatz her falsch.

Eine Insulinbehandlung schont die β-Zellen und behebt den Insulinmangel an den Zielzellen.

Die Insulinresistenz jedoch kann durch eine Insulinbehandlung nicht gebessert werden. Das hier vorgelegte Konzept mit den genannten Substanzen bietet jetzt die Möglichkeit, in die Pathogenese der Insulinresistenz gezielt einzugreifen.

### 4. Darstellung der Lösung

### 4.1. Zeichenerklärung

- Die Knoten der Kausalstruktur repräsentieren pathophysiologische Zustände.
- Die Kanten, d.h. die gerichteten Verbindungen der Form A ---> 8 und A ---| B bedeuten:
   Der pathophysiologische Zustand A erzeugt im ersten Fall eine Zunahme, im zweiten Fall eine Abnahme der Meßwerte des pathophysiologischen Zustands B.
- A↗ und A↙ bedeuten:
   Der pathophysiologische Zustand A befindet sich im ersten Fall oberhalb, im zweiten Fall unterhalb eines Normbereichs.

In der Fig.1 verwendete Abkürzungen:
- abgeb.: abgebaut
- aktiv.Leukoz.: aktivierte Leukozyten
- alpha2-adr.: alpha2-adrenerge
- Einwirk.: Einwirkung
- Endoth.Fktn.: Endothel-Funktion
- Erschöpf.: Erschöpfung
- G-Prot.: G-Proteine
- Gefäßw.: Gefäßwand
- HpHb: Haptoglobin-Hämoglobin-Komplex
- InsEintrsp.: Insulineintransport
- InsRez.ph.: Insulinrezeptor phosphoryliert
- InsWkg.: Insulinwirkung
- Leuk.Mediat.: Leukozyten-Mediatoren
- Leukoz.: Leukozyten
- Mediat.: Mediatoren
- Mikrozirk.: Mikrozirkulation
- Mukosaepith.: Mukosaepithel
- NO: Stickoxid
- PDDM verst.: Permeabilitätsdefekt der Dünndarmmukosa wird verstärkt
- periph.: peripher
- Phosph.l.Myosink.: Phosphorylierung leichter Myosinketten
- PKC: Proteinkinase C
- prädiab.: prädiabetisch
- Psych.Streß: Psychischer Streß
- Rheol.: Rheologie
- Säugl.: Säuglinge
- Schädig.: Schädigung
- Sekr.Regul.: Sekretionsregulatoren
- Ser-Pos.: Serin-Position
- Thr-Pos.: Threonin-Position
- Transp.: Transport
- Ueberempf.geg.psych.: Ueberempfindlichkeit gegen psychische
- Vit.A: Vitamin A
- Zielz.: Zielzelle

### 4.2. Schaubild

### Ueberblick:

Der zentrale Punkt des Kausalablaufs ist die Inaktivierung der Insulinrezeptoren des Gefäßendothels. Dadurch wird der Insulintransport über das Endothel zu den Zielzellen vereitelt.

Es ist damit die Frage zu klären, welche Mechanismen zur Inhibierung der Insulinrezeptoren im Gefäßendothel führen. Es sind drei Ursachen zu nennen:
- Adrenalinwirkung mit Phosphorylierung des Insulinrezeptors an der Serin-Position.
- Glukosewirkung mit Aktivierung der Proteinkinase C und Phosphorylierung des Insulinrezeptors an der Threonin-Position.
- Einwirkung von Leukozyten-Mediatoren mit Aktivierung der Proteinkinase C und Phosphorylierung des endothelialen Insulinrezeptors an der Threonin-Position.

Damit erhebt sich vor allem die Frage, wie die letztgenannte pathologische Aktivierung entsteht. Hierzu wird ein Permeabilitätsdefekt der Dünndarmmukosa postuliert, der Schadstoffe passieren läßt. Die Schadstoffe schädigen das Mukosaepithel und die wandständigen Enzyme. Eindringende schwer phagozytierbare Schadstoffpartikel induzieren im peripheren Blut die Ausschüttung von Mediatoren aus Leukozyten, die auch am Endothel wirken.

Es werden somit drei Hypothesen eingesetzt:

### Hypothese 1

Ein Permeabilitätsdefekt der Dünndarmmukosa läßt Schadstoffe pssieren, die zu einer Entzündungsreaktion der Mukosa führen, mit einer Schädigung wandständiger Enzyme und weiterer Beeinträchtigung der Durchlässigkeit.

### Hypothese 2

Die im peripheren Blut erscheinenden Schadstoffpartikel sind infolge einer besonderen Gestalt schwer phagozytierbar und führen zu einer steten Aktivierung der Leukozyten.

### Hypothese 3

Die Leukozyten-Mediatoren bewirken in den Membranen der Gefäßendothelzellen eine Proteinkinase C-Aktivität, die Insulinrezeptoren phosphoryliert.

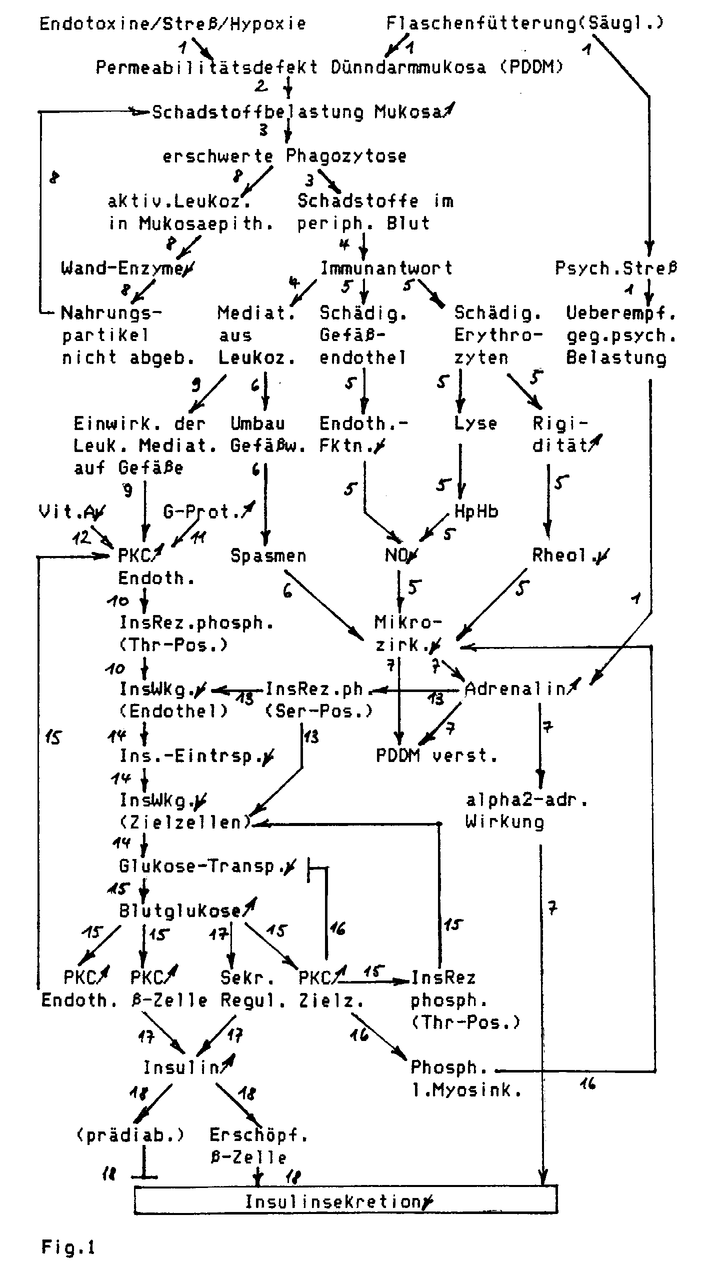

### 4.3. Erläuterung der Kausalverweise:

1
   Aufgrund der fühkindlichen Flaschenfütterung entsteht ein Permeabilitätsdefekt der Dünndarmmukosa (PDDM), der lebenslang fortbesteht. Ein solcher Permeabilitätsdefekt kann durch Endotoxine, Streß, intestinale Ischämie, Nahrungskarenz und bakterielle Fehlbesiedlung des Gastrointestinaltrakts verstärkt oder auch primär induziert werden.
   Die Flaschenfütterung bedeutet für den Säugling eine außerordentlich hohe Streßbelastung, so daß sich eine generelle Ueberempfindlichkeit gegenüber psychischen Belastungen etablieren kann.
2
   Der Chymus enthält nach der Dünndarmpassage auch bei Stoffwechselgesunden noch Protein-Polysaccharid-Komplexe, Gluten, Polypeptide, Grenzdextrine und Polysaccharide. Es wird angenommen, daß eine erhöhte Mukosapermeabilität ein gesteigertes Eindringen eben dieser Substanzen bewirkt, zusätzlich zu allergisierenden Nahrungsbestandteilen, Aromastoffen und Umweitgiften sowie bakteriellen Stoffwechselprodukten und Bakterien.
3
   Die nicht bereits in der Darmmukosa abgefangenen Schadstoffe gelangen über das Portalblut zur Leber, der zweiten Station der Fremdstoffbeseitigung. Als dritte Garde der Immunabwehr treten Blutzellen in Aktion, und erst danach erscheinen körperfremde Stoffe im peripheren Blut.
4
   Es wird vermutet, daß Grenzdextrine und Protein-Polysaccharid-Komplexe sowie weitere unidentifizierte Schadstoffpartikel zwar phagozytiert werden, daß sie aber aufgrund einer besonderen Gestalt den Verdauungsenzymen des Gastrointestinaltraktes und den Enzymen der Phagozyten keine Angriffsfläche bieten. Die Schadstoffe reichem sich in den Leukozyten an und bewirken dadurch
   - eine Herabsetzung der Phagozytosefähigkeit,
   - die Ausschüttung lysosomaler Faktoren in den extrazellulären Raum,
   - eine verstärkte Sekretion von Mediatoren.
5
   Die Auswirkungen der erschwerten Phagozytose sind Schädigungen des Gefäßendothels und der Erythrozyten.
   Der Angriff an den Membranlipiden der Erythrozyten mindert die Formbarkeit, eine Verringerung der Fließeigenschaften des Blutes ist die Folge. Fortgesetzte schädigende Einflüsse führen zur Hämolyse.
   Haptoglobin-Hämoglobin-Komplexe (HpHb), freies Hämoglobin und Bilirubin treten auf.
   Endothelschädigungen sind die Ursache für unzureichende Stickoxidfreisetzung, die zudem noch durch Haptoglobin-Hämoglobin-Komplexe beeinträchtigt wird.
6
   Bei verschiedenen Immunprozessen zeigen sich am Gefäßendothel zelluläre Infiltrationen der Intima und der Subintima mit Makrophagen, Monozyten, Granulozyten und Mastzellen.
   Die Folge sind akute und chronische Umbauprozesse der Gefäßwand, die zu lokalen Konstriktionen und Spasmen führen können.
7
   Die reduzierte Mikrozirkulation mit den damit verbundenen Hypoxiezuständen erhöht Adrenalin und damit die alpha2-adrenerge Insulinsekretionshemmung.
   Es besteht die Möglichkeit, daß überhöhte Adrenalinspiegel eine Minderung der intestinalen Barrierefunktion bewirken.
   Hypoxie, intestinale Ischämie verschlechtern die Integrität der Dünndarmmukosa.
8
   Aktivierte Leukozyten in der Dünndarmmukosa schädigen das Mukosaepithel und die lokalisierten wandständigen Enzyme, z.B. die (1--->6)Glukosidase, so daß infolge einer mangelnden Aktivität der Wand-Enzyme ein unvollständiger Nährstoffabbau stattfindet, der die Menge potentieller Schadstoffe erhöht.
9
   Die von Schadstoff-belasteten Leukozyten sezernierten Mediatoren verfolgen den Zweck, weitere Leukozyten zu aktivieren. Dies geschieht z.B. mit Hilfe von Leukotrien B4 (LTB4) und 5-Hydroxyeicosatetraensäure (5-HETE).
   Auch Diacylglycerin (DAG) kann im Blut nachgewiesen werden.
   Da die Aktivierung der Leukozyten über die Bildung von Inositoltriphosphat (IP3) und Diacylglycerin (DAG) mit nachfolgender Aktivierung der Proteinkinase C (PKC) erfolgt, ist es denkbar, daß die genannten Metaboliten auch in dem in unmittelbarer Nähe befindlichen Endothel den DAG-PKC-Weg anstoßen.
   Zusammenfassend wird formuliert:
   a.)
      Enzymatisch schwer aufschließbare Schadstoffpartikel verursachen eine fortgesetzte Aktivierung der Leukozyten. In besonderem Verdacht stehen Grenzdextrine und Protein-Polysaccharid-Komplexe aus Poaceae- und Solanaceae-Arten sowie Bakterien- und Zelltrümmer aus der zum Teil suizidalen Phagozytose der Leukozyten in der Darmmukosa.
   b.)
      Von Leukozyten sezernierte Mediatoren zur PKC-Aktivierung wirken in den Membranen von benachbarten Leukozyten und Gefäßendothelzellen.
10
   Durch PKC wird der Insulinrezeptor des Gefäßendothels an der Threonin-Position, Aminosäure 1348, phosphoryliert und so inaktiviert.
   Dadurch wird der Insulinrezeptorkomplex gehindert, gebundenes Insulin durch die Endothelzelle hindurch zu transportieren und an der kontralateralen Plasmamembran wieder freizusetzen. Das Insulinmolekül kann dann den Insulinrezeptor der metabolisch relevanten Zielzelle, z.B. der Muskel- oder Fettzelle nicht erreichen.
11
   Eine erhöhte Aktivierung der Phospholipase C wäre durch eine vermehrte Membranassoziation der Signal-vermittelnden G-Proteine möglich.
   Die verstärkte Membranassoziation der G-Proteine wiederum bildet sich als Folge einer intensiveren Prenylierung.
   Die Steigerung der Prenylierung könnte verursacht sein durch eine vermehrte Cholesterinzufuhr mit Hemmung der Squalen-Synthase und daraus sich ergebender Begünstigung der Prenyl-Transferasen.
   Exkurs:
   Das frühe Eingreifen in den Mevalonat-Stoffwechsel durch HMG-CoA-Reduktase-Hemmer (HCRH) stoppt neben der Cholesterinerzeugung vermutlich auch die Prenylierungen. Die Abnahme der Prenylierung ist für Ubiquinon nachgewiesen. Zum Nachweis dieses Effekts für G-Proteine verwendet man, daß die Signalübermittlung zur Aktivierung von Makrophagen mit Hilfe von G-Proteinen bewerkstelligt wird.
   Bei der Hemmung der extrahepatischen HMG-CoA-Reduktase durch HCRH sind somit zwei Effekte von Bedeutung:
   a. Hemmung der Prenylierung von G-Proteinen.
   b. Verringerung der LDL-Oxidation durch Makrophagen. Da oxidiertes LDL die endotheliale NO-Synthetase mindert, ist durch HCRH eine Verbesserung der endothelialen NO-Synthetase zu erwarten.
12
   Eine Inhibition des Insulinsignals wird auch durch eine pathologische Plasmamembranzusammensetzung erzeugt. Ein Vitamin A-Mangel, wie er bei Diabetes infolge ungenügender Carotinspaltung auftritt, könnte hier beteiligt sein.
13
   Insulinrezeptoren werden durch Katecholamine inhibiert. Es geschieht durch die Phosphorylierung der β-Untereinheit des Insulinrezeptors an der Serin-Position, Aminosäuren 1305 und 1306.
14
   Durch die Inaktivierung der Insulinrezeptoren des Gefäßendothels sind die Insulinrezeptoren der Zielzellen unterversorgt, der Glukose-Eintransport ist gestört. Hierbei steuern die Insulinrezeptoren den Glukosetransport vermutlich über die Aktivierung von Phospholipase C (PLC).
15
   Der verminderte Glukose-Eintransport in die Zielzellen läßt die Blutglukose ansteigen. Bei hohen Blutglukosekonzentrationen tritt eine Verringerung der Insulin-Signaltransduktion auf.
   Es ist anzunehmen, daß das durch Glukose reduzierte Insulinsignal die Folge einer Aktivierung der Proteinkinase C ist.
16
   Die bei hohen Blutglukosekonzentrationen aktivierte Proteinkinase C bewirkt gleichzeitig eine Verstärkung des Glukosetransports, so daß bei erhöhtem Glukosespiegel ein Glukose-Eintransport auch insulinunabhängig bewerkstelligt werden kann.
   Außerdem phosphoryliert PKC die leichten Myosinketten der glatten Muskulatur. Durch diese Phosphorylierung wird die glatte Muskulatur zur Kontraktion angeregt.
17
   Die erhöhte Blutglukose ist an der pankreatischen B-Zelle Ursache für die Steigerung der Insulinsekretion, die über die Glukose-induzierte Erhöhung der PKC-Aktivität und über weitere Sekretionsregulatoren gesteuert wird.
18
   Durch den fortwährenden Sekretionsreiz erschöpft die B-Zelle; der prädiabetische Hyperinsulinismus geht in einem langdauernden Prozeß in einen Hypoinsulinismus über.

## Patentansprüche

1. Zur Therapie und Prophylaxe von Erkrankungen, die eine Insuliresistenz aufweisen, insbesondere des Diabetes mellitus, werden in beliebiger Auswahl und Kombination die Flavonoidglykoside
- Hesperidin,
- Rutosid,
- Rutosid-Trihydrat,
die Glykoside der Anthocyanidine
- Pelargonidin,
- Cyanidin,
- Malvidin,
die erprobten Gerbstoffdrogen
- Folia Theae von Camellia sinensis,
- Fructus Myrtilli von Vaccinium myrtillus,
- Fructus castaneae von Castanea sativa
sowie die wenig erprobten und daher mit Risiko behafteten Gerbstoffdrogen
- Catechu von Acacia catechu,
- Herba Agrimoniae von Agrimonia eupatoria,
- Herba Alchemillae von Alchemilla xanthochlora,
- Folia castaneae von Castanea sativa,
- Herba Fragariae von Fragaria vesca,
- Cortex Hamamelidis von Hamamelis virginiana,
- Folia Juglandis von Juglans regia,
- Radix Ratanhiae von Krameria triandra,
- Radix Tormentillae von Potentilla erecta,
- Herba Anserinae von Potentilla anserina,
- Kino von Pterocarpus marsupium,
- Gallae von Quercus infectoria,
- Cortex Quercus von Quercus robur,
- Folia Rubi fruticosi von Rubus fruticosus,
- Herba Sanguisorbae von Sanguisorba officinalis,
- Gambir Catechu von Uncaria Gambir
angewendet, dadurch gekennzeichnet, daß durch diese Stoffe in bekannter Weise ein Permeabilitätsdefekt der Dünndarmmukosa gebessert werden kann, so daß dadurch ein pathophysiologischer Anfangszustand eines neu entwickelten pathogenetischen Konzepts der Insulinresistenz verbessert wird.

2. Zur Therapie und Prophylaxe von Erkrankungen, die eine Insuliresistenz aufweisen, insbesondere des Diabetes mellitus, werden in beliebiger Auswahl die Mikroorganismen
- Lactobacillus acidophilus,
- Lactobacillus bifidus und
- Saccharomyces boulardii
angewendet, dadurch gekennzeichnet, daß sich durch diese Stoffe in bekannter Weise eine physiologischere Bakterienbesiedlung des Darms einstellt, die Art und Menge der in die Darmmukosa eindringenden Schadstoffe günstig beeinflußt, wodurch wiederum ein Anfangszustand eines neu entwickelten pathogenetischen Konzepts der Insulinresistenz gebessert wird.

3. Verwendung der Stoffe gemäß Anspruch 2 mit der Möglichkeit, die Liste der einsetzbaren Substanzen zu erweitern durch
- Saccharomyces cerevisiae,
appliziert z.B. als sogenannte Bäckerhefe oder Bierhefe.

4. Zur Therapie und Prophylaxe von Erkrankungen, die eine Insuliresistenz aufweisen, insbesondere des Diabetes mellitus, werden
- Nahrungsmittel mit reduziertem Amylopektingehalt, inbesondere Amylopektin-reduzierte Konfitüren,
die Pankreas-Verdauungsenzyme
- Trypsin,
- Chymotrypsin,
- Carboxipeptidase A und B,
- alpha-Amylase,
möglichst alle Enzyme gleichzeitig zu den Mahlzeiten, sowie in beliebiger Auswahl die in Pflanzen enthaltenen Proteasen
- Bromelain,
- Papain und
- Carboxipeptidasen
angewendet, dadurch gekennzeichnet, daß sich durch diese Stoffe in bekannter Weise eine bessere Situation hinsichtlich der Verdauung von Protein-Polysaccharid-Komplexen, Polypeptiden und Amylopektin einstellt, die Art und Menge der in die Darmmukosa eindringenden Schadstoffe günstig beeinflußt, wodurch ein Anfangszustand eines neu entwickelten pathogenetischen Konzepts der Insulinresistenz gebessert wird.

5. Zur Therapie und Prophylaxe von Erkrankungen, die eine Insuliresistenz aufweisen, insbesondere des Diabetes mellitus, werden die HMG-CoA-Reduktase-Hemmer
- Simvastatin,
- Lovastatin,
- Pravastatin und
- Fluvastatin
angewendet, bei gemessenem erhöhten Serumcholesterin eine der Substanzen mit Bevorzugung von Simvastatin, dadurch gekennzeichnet, daß sich durch diese Stoffe in bekannter Weise eine Erhöhung der NO-Synthetase und eventuell eine Senkung überhöhter G-Protein-Membranassoziationen einstellt, wodurch Zwischenzustände eines neu entwickelten pathogenetischen Konzepts der Insulinresistenz gebessert werden.

6. Zur Therapie und Prophylaxe von Erkrankungen, die eine Insuliresistenz aufweisen, insbesondere des Diabetes mellitus, wird der alpha2-Rezeptor-Antagonist
- Yohimbim
angewendet, dadurch gekennzeichnet, daß sich durch diesen Stoff in bekannter Weise an der β-Zelle eine Lockerung der alpha2-adrenergen Insulinsekretionshemmung und am Gefäßendothel eine Minderung der Katecholamin-induzierten Insulinrezeptorphosphorylierung einstellt, wodurch wiederum Zwischenzustände eines neu entwickelten pathogenetischen Konzepts der Insulinresistenz bessernd beeinflußt werden.

7. Zur Therapie und Prophylaxe von Erkrankungen, die eine Insuliresistenz aufweisen, insbesondere des Diabetes mellitus, werden in einer Art Schrotschuß-Strategie
- die Vitamine A, B1, B2, B6, B12, Folsäure, Nicotinamid, Pantothensäure, C, D, E, Biotin, Carnitin, alpha-Liponsäure, Rutosid, Hesperidin,
- die Elektrolyte Mg, Ca, K, Na
- sowie die Spurenelemente Fe, J, Zn, Cu, Mn, Mo, Se, Cr angewendet, dadurch gekennzeichnet, daß möglichst viele dieser Stoffe möglichst gleichzeitig eingesetzt werden, weiter dadurch gekennzeichnet, daß sich durch diese Substanzen eine Besserung einstellen könnte hinsichtlich
* einer Ernährungmangelsituation von Epithelzellen der Dünndarmmukosa und eines damit verbundenen Permeabilitätsdefekts, oder hinsichtlich
* einer Ernährungsmangelsituation der Leukozyten und eines damit verbunden Defekts der Phagozytosefähigkeit,
oder hinsichtlich
* einer unphysiologischen Bakterienbesiedlung des Gastrointestinaltraktes und einer damit verbundenen Bildung von Schadstoffen, oder hinsichtlich
* einer gestörten Funktion des Gefäßendothels,
so daß pathophysiologische Zustände des neu entwickelten pathogenetischen Konzepts der Insulinresistenz bessernd beeinflußt werden.

8. Verwendung der Stoffe gemäß Anspruch 1 bis 7 zur Erreichung milderer Verlaufsformen für die sogenannten diabetischen Spätschäden bzw. für die sogenannten diabetischen Begleiterkrankungen
- Mikro- und Makroangiopathie,
- Hypertonie,
- periphere Neuropathie,
- Nephropathie,
- nichtalkoholische Steatohepatitis,
- Polyarthritis und
- Herzrhythusstörungen,
da das neu entwickelte pathogenetische Konzept der Insulinresistenz pathophysiologische Zustände oder sogar Kausalketten aufweist, die auch in den pathogenetischen Konzepten der obigen Spätfolgen bzw. Begleiterkrankungen zu finden sind, so daß eine Besserung solcher gemeinsamer pathophysiologischer Zustände auch die Entwicklung der genannten Erkrankungen günstig beeinflußt.

9. Verwendung von Messungen
- einer verminderten Phagozytosefähigkeit von Monozyten und Granulozyten, und/oder
- einer verminderten Leukozytenanzahl im Serum, und/oder
- einer erhöhten Anzahl Cytotoxischer T-Lymphozyten im Serum, und/oder
- einer verminderten Konzentration von Serumtransferrin als Marker für einen sich entwickelnden Diabetes mellitus, oder als Marker für sich entwickelnde diabetische Begleiterkrankungen aus der Liste der Erkrankungen gemäß Anspruch 8, dadurch gekennzeichnet, daß die Leukozytenaktivitäten wesentlich sind für die Ausbildung charakteristischer pathophysiologischer Zustände in dem neuen pathogenetischen Konzept der Insulinresistenz.

10. Verwendung von Anspruch 9 dadurch ergänzt, daß zur Sicherung der Prognose eines Diabetes mellitus oder einer sogenannten diabetischen Begleiterkrankung beliebig viele der Hämolyseanzeichen
- Erhöhung des unkonjugierten Bilirubins,
- Erhöhung der LDH-Aktivität,
- Erhöhung des freien Serumhämoglobins,
- Erhöhung des Retikulozyten-Produktionsindexes und
- Erniedrigung des Serumhaptoglobins
verwendet werden, dadurch gekennzeichnet, daß eine Hämolyse u.a. durch die Aufnahme von Schadstoffen in den Erythrozyten oder durch die Phagozytoseaktivität der Leukozyten entsteht, und daß die Hämolyse dadurch an einer charakteristischen Stelle des neu entwickelten pathogenetischen Konzepts der Insulinresistenz lokalisiert ist.
